# EUROPÄISCHE PATENTSCHRIFT

(11) **EP 1 908 458 B1**
(45) Veröffentlichungstag und Bekanntmachung des Hinweises auf die Patenterteilung: **16.03.2011**
(21) Anmeldenummer: 07024046.0
(22) Anmeldetag: 10.09.2001
(51) Int. Cl.: A61K 31/02, A61P 23/00

(54) **Volatile Anästhesiemittel mit Xenon**
Volatile anaesthetic with xenon
Agent anesthésique volatile au xénon

(30) Priorität: 14.09.2000 DE 10045829
(43) Veröffentlichungstag der Anmeldung: 09.04.2008
(62) Teilanmeldung aus: 01965268.4
(73) Patentinhaber: Air Liquide Deutschland GmbH, 40235 Düsseldorf (DE)
(72) Erfinder: Copping, Arnold, 9111 Belsele (BE); Neu, Peter, 47228 Duisburg (DE); Horn, Nicola, 47559 Kranenburg (DE); Pilger, Carsten, 47509 Rheurdt (DE); Thoma, Klemens, 47800 Krefeld (DE); Rossaint, Rolf, 52074 Aachen (DE); Reyle-Hahn, Matthias, 14129 Berlin (DE); Hecker, Klaus E., 50126 Bergheim (DE); Baumert, Jan-Hinrich, 52062 Aachen (DE); Schucht, Fridtjof, 47803 Krefeld (DE)
(74) Vertreter: Kahlhöfer, Hermann

(56) Entgegenhaltungen:
- EP-A- 0 523 315
- WO-A-00/04909
- WO-A-00/53192
- US-A- 4 113 883
- F. GIUNTA ET AL.: "Xenon: A review of its anaesthetic and pharmacological properties" APPLIED CARDIOPULMONARY PATHOPHYSIOLOGY, Bd. 6, Nr. 2, 1996, Seiten 95-103, XP002188236

## Beschreibung

Die Erfindung betrifft ein volatiles Anästhesiemittel, das Xenon enthält oder in Kombination mit Xenon eingesetzt wird.

Zu den volatilen Anästhesiemitteln zählen Methoxyfluran, Halothan, Enfluran, Isofluran, Sevofluran und Desfluran. Methoxyfluran wird aufgrund gesundheitlicher Risiken nicht mehr eingesetzt.

Xenon wird schon seit vielen Jahren als Inhalationsanästhesiemittel eingesetzt. Eine Übersicht über die anästhetischen und pharmakologischen Eigenschaften des Xenon findet sich in F. Giunta et al., "Xenon: a review of its anaesthetic and pharmacological properties", Applied Cardiopulmonary Pathophysiology 00: 1-9, 1996.

Als ein Maß der anästhetischen Potenz eines Anästhesiemittels wurde der sogenannte MAC-Wert (minimal alveolar concentration) eingeführt. Der MAC-Wert ist ein Richtwert in der Anästhesie. Die real erforderliche Anästhesiemittelkonzentration ist von Patient zu Patient verschieden und hängt von vielen Parametern wie u. a. dem Lebensalter ab. Die erforderliche Anästhesiemittelkonzentration schwankt daher gewöhnlich im Bereich des 0,7 bis 1,3 fachen Wertes des MAC-Wertes. Nach derzeitigem Kenntnisstand liegt der MAC-Wert beim Menschen für Xenon bei 71 Vol.-% Xenon.

Die bekannten volatilen Inhalationsanästhesiemittel haben eine Reihe von Nachteilen. So haben diese Mittel Blutdruck senkende Wirkung, werden im Körper nur allmählich abgebaut oder über Leber und Niere ausgeschieden und haben in der Regel unerwünschte Nebenwirkungen.

Der Erfindung liegt die Aufgabe zugrunde, ein Mittel für die Anästhesie bereitzustellen, das weniger Nachteile aufweist.

Überraschend wurde gefunden, daß bei einer Verabreichung von einem volatilen Anästhesiemittel mit gasförmigem Xenon die Nachteile bei dem Einsatz von volatilen Anästhesiemitteln deutlich verringert werden.

Gegenstand der Erfindung ist somit ein Anästhesiemittel mit den in Anspruch 1 beschriebenen Merkmalen.

Das Anästhesiemittel enthält neben Sauerstoff als eine Komponente Xenon oder ein xenonhaltiges Gas und als weitere Komponente ein volatiles Anästhesiemittel, wobei die Komponenten zur gleichzeitigen, getrennten oder zeitlich abgestuften Anwendung bei der Anästhesie eingesetzt werden. Das Anästhesiemittel, das besonders bevorzugt in Form der getrennten Komponenten in abgestimmter Weise zur Anästhesie eingesetzt wird, wird daher auch als Kombinationsanästhesiemittel oder als Kombinationsinhalationsanästhesiemittel bezeichnet. Das Anästhesiemittel oder seine Komponenten sind bei Gebrauch gasförmig. Das Kombinationsinhalationsanästhesiemittel wird vorzugsweise unmittelbar vor dem Gebrauch hergestellt, indem die aktuell einzusetzenden Inhalationsanästhesiemittelkomponenten, Sauerstoff und gegebenenfalls ein Gas, vorzugsweise ein Gas mit inerten Eigenschaften (z. B. ein Inertgas) zu einem atembaren Gas gemischt werden.

Ein weiterer Gegenstand der Erfindung ist die Verwendung von Sauerstoff Xenon in eines konzentration in einem Bereich von 10 bis 50 Vol-% und einem oder mehreren volatilen Anästhesiemitteln als Komponenten zur Herstellung eines Kombinationsanästhesie in einen konzentration in einem mereich von 10 61 50 Vol.-%. mittels zurgleichzeitigen, getrennten oder zeitlich abgestuften Anwendung der Komponenten bei der Anästhesie.

Das Kombinationsanästhesiemittel wird beim Menschen oder bei Säugetieren eingesetzt.

Als volatiles Anästhesiemittel werden z. B. Halothan, Enfluran, Isofluran, Sevofluran oder Desfluran verwendet. Die Komponente des volatilen Anästhesiemittels bei dem Kombinationsanästhesiemittel kann auch ein Gemisch von zwei oder mehreren volatilen Anästhesiemitteln sein. Bevorzugt wird der Einsatz eines einzelnen volatilen Anästhesiemittels in dem Kombinationsanästhesiemittel.

Das Anästhesiemittel wird vorzugsweise während des Gebrauches durch Zudosierung von Xenon oder von einem xenonhaltigen Gas und einem volatilen Anästhesiemittel zu einem Gasstrom, der aus Sauerstoff besteht oder für die Atmung in ausreichender Menge Sauerstoff enthält, erzeugt. Die Zudosierung der Anästhesiemittelkomponenten erfolgt beispielsweise in einen Beatmungsschlauch eines Patienten. Das erzeugte Gasgemisch oder das Kombinationsanästhesiemittel enthalten Xenon in einer Konzentration im Bereich von 10 bis 50 Vol.-%.

Die zur Anästhesie erzeugten Gasgemische können neben Sauerstoffs, Xenon und dem volatilen Anästhesiemittel ein oder mehrere Gase oder bei Körpertemperatur und Normaldruck gasförmige Stoffe enthalten. Zusätzliche Gase sind beispielsweise ein Inertgas wie Stickstoff oder Edelgase (z. B. Helium, Neon, Argon, Krypton). Die Beimischung eines oder mehrerer inerter oder sich inert verhaltenden Gase kann sehr vorteilhaft sein, wenn wenig Xenon in den Körper gebracht werden soll. Dies kann zum Beispiel bei der Beendigung einer Narkose der Fall sein.

Das erzeugte Gasgemisch oder das Kombinationsanästhesiemittel enthalten das volatile Anästhesiemittel vorzugsweise in einer Konzentration unterhalb des entsprechenden MAC-Wertes, beispielsweise unterhalb von 90 % des entsprechenden MAC-Wertes, vorzugsweise unterhalb von 75 % des entsprechenden MAC-Wertes, besonders bevorzugt unterhalb von 50 % des entsprechenden MAC-Wertes, insbesondere unterhalb von 25 % des entsprechenden MAC-Wertes.

Bei Patienten mit Leber- oder Nierenschäden wird vorzugsweise Isofluran, bei Patienten mit einem Herzleiden oder krankem Herzen wird vorzugsweise Enfluran und in normalen Fällen wird vorzugsweise Isofluran, Sevofluran oder Desfluran als volatiles Anästhesiemittel in dem Kombinationsanästhesiemittel eingesetzt. Für ein schnelles Aufwachen des Patienten nach der Narkose wird vorzugsweise Sevofluran oder Desfluran eingesetzt. Bei Kindern werden vorzugsweise Sevofluran oder Halothan als volatiles Anästhesiemittel in dem Kombinationsanästhesiemittel eingesetzt.

Die volatilen Anästhesiemittel Halothan, Enfluran, Isofluran, Sevofluran oder Desfluran werden vorzugsweise in den folgenden Konzentrationsbereichen im Kombinationsanästhesiemittel oder Gasgemisch beim Menschen eingesetzt: von 0,2 bis 0,75 Vol.-% bei Halothan, von 0,6 bis 1,6 Vol.-% bei Enfluran, von 0,5 bis 1,15 Vol.-% bei Isofluran, von 0,9 bis 2,0 Vol.-% bei Sevofluran und von 3,6 bis 6,0 Vol.-% bei Desfluran (Werte extrapoliert von Versuchen mit Schweinen).

Zur Anästhesie werden Gasgemische eingesetzt, bestehend aus 10 bis 50 Vol.-% Xenon, einem volatilen Anästhesiemittel, Sauerstoff und gegebenenfalls einem oder mehreren weiteren Gasen, wobei die Anteile aller Komponenten des Gasgemisches zusammen 100 Vol.-% ergeben. Der Sauerstoffgehalt in dem Gasgemisch beträgt in der Regel mehr als 20 Vol.-%, vorzugsweise um 30 Vol.-%. In besonderen Fällen, insbesondere in momentanen Notsituationen, können zeitweise Sauerstoffkonzentrationen von mehr als 40 Vol.-% im Gasgemisch (Atemgas), insbesondere um 50 Vol.-% oder mehr als 50 Vol.-%, je nach Anwendungsfall, vorteilhaft sein. Eine ständig erhöhte Sauerstoffkonzentration im Gasgemisch ist vorteilhaft insbesondere zur Anästhesie von Patienten mit pulmonalen Erkrankungen oder bei Operationen mit großem Blutverlust. In solchen Fällen werden beispielsweise Gasgemische mit Xenon und volatilem Anästhesiemittel mit einem Anteil von mindestens 30 Vol.-% Sauerstoff (FiO₂ ≥0,3; FiO₂ = fractional inspired oxygen tension) oder mindestens 50 Vol.-% Sauerstoff (FiO₂ ≥ 0,5), je nach Anwendungsfall, eingesetzt.

In der Regel werden Anteile der Gaskomponenten im erzeugten Gasgemisch während einer Narkose verändert, das heißt die Anästhesiemittelkomponenten oder eine der Anästhesiemittelkomponenten werden nicht konstant oder nur zeitweise konstant gehalten. Die Anteile der Anästhesiemittelkomponenten werden z.B. einzeln oder paarweise stufenweise oder stetig verändert. Eine Komponente wie Xenon odervolatiles Anästhesiemittel kann über ein Zeitintervall während der Narkose auch ganz ausgesetzt werden.

Beispiele von Gasgemischen (Konzentrationsstufen), zwischen denen in einer Narkose gewechselt werden kann (z.B. von einem Gasgemisch mit 30 Vol.-% Sauerstoff zu einem Gasgemisch mit 50 Vol.-% Sauerstoff), sind in den Tabellen 1 bis 5 aufgeführt. Die Gasgemische werden in der Regel in der Weise hergestellt, daß zu einem Trägergasstrom aus reinem Xenon die Volumenanteile der übrigen Komponenten ( von Sauerstoff und einem volatilen Anästhesiemittel) zudosiert werden. Die Gasdosierung erfolgt in der Regel mit einem Anästhesiegerät. Eine Narkose wird in vielen Fällen mit einem Injektionsanästhesiemittel wie Propofol eingeleitet und mit dem Kombinationsinhalationsanästhesiemittel aufrechterhalten. Beispielsweise wird ein Gasgemisch aus 70 Vol.-% Xenon und 30 Vol.-% Sauerstoff zu Beginn der Inhalationsnarkose verabreicht. Bei höherer Sauerstoffdosierung wird z. B. auf eine andere Sauerstoffkonzentrationsstufe, wie in den Tabellen 1 bis 5 gezeigt, umgestellt, wobei die Narkosetiefe durch Veränderung der Konzentration des volatilen Anästhesiemittels in den aufgeführten Bereichen eingestellt wird. Im Verlauf der Narkose wird auf eine andere Sauerstoffkonzentrationsstufe oder die ursprüngliche Sauerstoffkonzentrationsstufe umgestellt.

**Tabelle 1: Beispiele von Xenon/Isofluran/Sauerstoff-Gasgemisehen (für die Anästhesie des Menschen)**

| Sauerstoff / vol.-% | Isofluran /vol.-% Bereich: von/bis | Xenon / vol.-% (ungefährer Wert) | |
|---|---|---|---|
| 25 | 0/0 | 75 | (nicht erfindungsgemäß) |
| 30 | 0/0 | 70 | " |
| 35 | 0/0,15 | 65 | " |
| 40 | 0,15/0,3 | 60 | " |
| 45 | 0,25/0,38 | 55 | " |
| 50 | 0,35/0,45 | 50 | (erfindungsgemäß) |
| 55 | 0,4/0,53 | 45 | " |
| 60 | 0,45/0,6 | 40 | " |
| 70 | 0.53/0,85 | 30 | " |
| 80 | 0.85/1 | 20 | " |
| 90 | 1/1,15 | 10 | " |

**Tabelle 2: Beispiele von Xenon/Enfluran/Sauerstoff-Gasgemischen (für die Anästhesie des Menschen)**

| Sauerstoff / vol.-% | Enfluran / vol.-% Bereich: von / bis | Xenon / vol.-% (ungefährer Wert) | |
|---|---|---|---|
| 25 | 0/0 | 70 | (nicht erfindungsgemäß) |
| 30 | 0/0 | 75 | " |
| 35 | 0/0,3 | 65 | " |
| 40 | 0,3/0,6 | 60 | " |
| 45 | 0,5/0,8 | 55 | " |
| 50 | 0,8/1,0 | 50 | (erfindungsgemäß) |
| 55 | 1,0/1,2 | 45 | " |
| 60 | 1,2/1,3 | 40 | " |
| 70 | 1,3/1,4 | 30 | " |
| 80 | 1,4/1,5 | 20 | " |
| 90 | 1,5/1,6 | 10 | " |

**Tabelle 3: Beispiele von Xenon/Desfluran/Sauerstoff-Gasgemischen (für die Anästhesie des Menschen)**

| Sauerstoff / vol.-% | Desfluran / vol.-% Bereich: von /bis | Xenon / vol.-% (ungefährer Wert) | |
|---|---|---|---|
| 25 | 0/0 | 75 | (nicht erfindungsgemäß) |
| 30 | 0/0 0 | 70 | " |
| 35 | 0,5/0,8 | 65 | " |
| 40 | 0,8/1,0 | 60 | " |
| 45 | 1,0/1,5 | 55 | " |
| 50 | 1,5/2,0 | 50 | (erfindungsgemäß) |
| 55 | 2,0/2,5 | 45 | " |
| 60 | 2,5/3,0 | 40 | " |
| 70 | 3,0/4,0 | 30 | " |
| 80 | 4,0/5,0 | 20 | " |
| 90 | 5,0/6,0 | 10 | " |

**Tabelle 4: Beispiele von Xenon/Sevofturan/Sauerstoff-Gasgemischen (für die Anästhesie des Menschen)**

| Sauerstoff / vol.-% | Sevofluran / vol.-% Bereich: von / bis | Xenon / vol.-% (ungefährer Wert) | |
|---|---|---|---|
| 25 | 0/0 | 75 | (nicht erfindungsgemäß) |
| 30 | 0/0 0 | 70 | " |
| 35 | 0,4/0,6 | 65 | " |
| 40 | 0,6/0,9 | 60 | " |
| 45 | 0,9/1,1 | 55 | " |
| 50 | 1,1/1,3 | 50 | (erfindungsgemäß) |
| 55 | 1,3/1,5 | 45 | |
| 60 | 1,5/1,7 | 40 | |
| 70 | 1.7/1,8 | 30 | " |
| 80 | 1,8/1,9 | 20 | " |
| 90 | 1,9/2,0 | 10 | " |

**Tabelle 5: Beispiele von Xenon/Hatothan/Sauerstoff-Gasgemischen (für die Anästhesie des Menschen)**

| Sauerstoff/vol.-% | Halothan/vol.-% Bereich: von/bis | Xenon/vol.-%(ungefährer Wert) | |
|---|---|---|---|
| 25 | 0/0 | 75 | (nicht erfindungsgemäß) |
| 30 | 0/0 | 70 | " |
| 35 | 0/0,1 | 65 | " |
| 40 | 0,1/0,15 | 60 | " |
| 45 | 0,15/0,22 | 55 | " |
| 50 | 0,22/0,35 | 50 | (erfindungsgemäß) |
| 55 | 0,35/0,42 | 45 | " |
| 60 | 0,42/0,5 | 40 | " |
| 70 | 0,5/0,6 | 30 | " |
| 80 | 0,6/0,7 | 20 | " |
| 90 | 0,7/0,75 | 10 | " |

Bei konstanter Sauerstoffkonzentration (z. B. 30 Vol.-% Sauerstoff) können verschiedene Xenonkonzentrationen durch Dosierung eines zusätzlichen Inertgases wie Stickstoff eingestellt werden, .

Vorteilhaft wird in dem Kombinationsanästhesiemittel oder Gasgemisch der Anteil von Xenon und volatilem Anästhesiemittel so bemessen, daß der Blutdruck des Patienten während der Anästhesie im wesentlichen konstant bleibt oder daß kritische Blutdruckwerte bei einem Patienten während der Anästhesie vermieden werden. Dies wird nach derzeitigem Kenntnisstand bei Xenon-Konzentrationen im Bereich von 30 bis 55 Vol.-% in Kombination mit 0,25 bis 0,85 Vol.-% Isofluran, 0,5 bis 1,4 Vol.-% Enfluran, 1,0 bis 4,0 Vol.-% Desfluran, 0,9 bis 1,8 Vol.-% Sevofluran oder 0,15 bis 0,6 Vol.-% Halothan erreicht.

Die kombinierte Verwendung von Xenon und einem volatilen Anästhesiemittel bringt große Vorteile bei der Anästhesie gegenüber der Verwendung des einzelnen Anästhesiemittels. Bei dem Kombinationsinhalationsanästhesiemittel ist ein Sauerstoffgehalt im Beatmungsgas von mehr als 29 Vol.-% möglich (MAC von Xenon bei 71 % beim Menschen). Kritische Operationen, die eine höhere Sauerstoffzufuhr bei der Beatmung des Menschen erfordern, sind daher möglich, wobei die Vorteile bei der Verabreichung von Xenon weitgehend genutzt werden. Die volatilen Anästhesiemittel werden in solchen Mengen einsetzbar, wo die Nebenwirkungen der volatilen Anästhesiemittel nicht mehr in dem bekannten Maße auftreten. Insbesondere erhält der Anästhesist bei dem Kombinationsanästhesiemittel eine höhere Flexibilität in der Steuerung einer Narkose. Überraschenderweise läßt sich durch Verabreichung von Xenon die in der Regel mehr oder weniger ausgeprägte Absenkung des Blutdruckes bei der Anästhesie gegensteuern und sogar weitgehend kompensieren. Durch gezielte Dosierung von Xenon in das Atemgas wird bei Einsatz eines volatilen Anästhesiemittels eine möglicherweise gefährliche Blutdrucksenkung verhindert.

Beispielsweise gibt es einen additiven anästhetischen Effekt von inhaliertem Xenon und Isofluran, der es dem Anästhesisten erlaubt, die Anästhesie mit Xenon in einer schnellen, einfachen und billigen Weise zu ergänzen, wenn ein höherer Sauerstoffbedarf des Patienten besteht. Z.B. bei einem FiO₂ 0,5 ergibt sich eine Xenon-Konzentration von 50 Vol.-%, der MAC von Isofluran wird um etwa 30 % gesenkt. Dies erlaubt eine Senkung der Isofluran-Dosis, was die Nebenwirkungen reduziert und zu einer sichereren Anästhesietechnik führt. Die kombinierte Dosierung von Xenon und einem volatilen Anästhesiemittel zur Anästhesie ist besonders vorteilhaft bei Patienten mit kardiopulmonalen Erkrankungen und bei Operationen mit zu erwartendem hohen Blutverlust.

Das eingesetzte Xenon-Gas hat im allgemeinen die natürliche Isotopenzusammensetzung. Die Isotopenzusammensetzung des Xenons kann sich von der natürlichen Isotopenzusammensetzung unterscheiden.

Das Xenon-Gas wird vorzugsweise in hoher Reinheit, wie für medizinische Gase üblich, eingesetzt. Das Xenon-Gas dient als reines Gas oder im Gemisch mit anderen Gasen als Komponente von einem Kombinationsinhalationsanästhesiemittel oder Kombinationsinhalationsnarkosemittel zur gleichzeitigen, getrennten oder zeitlich abgestuften Anwendung mit den anderen Anästhesiemittelkomponenten bei der Anästhesie.

Gasförmiges Xenon wird im allgemeinen als komprimiertes, reines Gas in Druckgasbehältem wie Druckgasflaschen oder Druckdosen bereitgestellt. Xenon kann auch in einem Behälter als verflüssigtes Gas oder in kälteverfestigter Form bereitgestellt werden. Volatile Anästhesiemittel werden im allgemeinen als Flüssigkeiten bereitgestellt.

Volatile Anästhesiemittel und Xenon werden als Kombinationsanästhesiemittel beispielsweise mit Minimal flow-Anästhesiegeräten oder sogenannten Closed loop-Anästhesiegeräten, z.B. mit dem Gerät "Physioflex" von der Firma Dräger (Lübeck, Deutschland), verabreicht.

### Beispiele

### 1. Durchführung der Narkose mit Halothan-Xenon am Schwein Methode

Nach Genehmigung der zuständigen Tierschutzbehörde (Bez.-Regierung Köln, AZ: 23.203.2-AC 38, 27/99) wurde die Bestimmung des MAC-Wertes an 10 weiblichen Schweinen einer deutschen Landrasse mit einem Gewicht vom 28,6 - 35,8 kg (im Mittel 31,5 kg) bestimmt. Bei Aufnahme der Versuchstiere in das Institut für Versuchstierkunde der RWTH Aachen wurden alle Tiere von Tierärzten des Instituts eingehend untersucht. Diese Untersuchung umfaßte unter anderem das Verhalten des Tieres, die Körperhaltung, Gliedmaßen, Haut und Haare und die Schleimhäute. Herz und Lungen wurden auskultiert. Bei keinem Tier zeigten sich krankheitsbedingte Veränderungen. Die Körpertemperatur der Tiere lag nach Aufnahme zwischen 37,2 und 39,2 °C (im Mittel bei 38,2°C). Nach einer Überwachungsperiode von mindestens fünf Tagen und Ernährung mit einer Standardtiemahrung für Schweine, wurden die Tiere in den Versuch gebracht.

### Tiervorbereitung und Monitoring

Nach einer Prämedikation von 3 mg/kg Azerperon i.m. (intramuskulös) wurde 20 min. später eine Venenverweilkanüle in eine Ohrvene gelegt. Die Einleitung der Narkose erfolgte mit Propofol in 2 mg/kg KG. Die Intubation erfolgte ohne Muskelrelaxanz mit einem 7.5 mm Woodbridge-Tubus. Im weiteren Verlauf wurde eine arterielle Kanüle percutan in die Ohrarterie zur blutigen Blutdruckmessung und div. Blutabnahmen gelegt. Die Narkose wurde in dieser Phase mit Boli von 20 mg/kg KG Propofol fortgesetzt. Die kumulativen Dosen lagen zwischen 170 und 500 mg (im Mittel 276 mg). Die Tiere wurden kontrolliert beatmet, so daß das exspiratorische CO₂ kontinuierlich zwischen 40-45 mmHg lag. Alle Tiere wurden mit einem Inspirations/Exspirationsverhältnis von 1:1 und einem positiv end-exspiratorischen Druck (P EEP) von +3 cmH₂O beatmet. Als Narkosegerät diente ein Dräger PhysioFlex. Die Köpertemperatur wurde mittels einer Heizdecke (warm-touch, Mallinckrodt Medical, Ireland) auf einer Temperatur zwischen 38,0 °C und 39,5 °C konstantgehalten. Kontinuierlich wurden MAP (mittlerer arterieller Druck; mean arterial blood pressure), HR (Herzfrequenz; heart rate), Temperatur gemonitort (AS/3 compact, Datex-Engström, Helsinki/Finnland). Bei Applikation des supramaximalen Stimulus wurden die jeweils aktuellen Werte notiert. Die in- und exspiratorische Konzentration des Halothans wurde mittels Infrarot Spektroskopie (AS/3 compact, Datex-Engström, Helsinki, Finnland) die inspiratorische Xenon-Konzentration wurde mittels Infrarot Spektroskopie mit mit Hilfe des PhysioFlex (Physio á Dräger company, Lübeck Germany) gemonitort. Aufgrund der pharmacokinetischen Eigenschaften des Edelgases Xenon, ist von einer sehr raschen Äquilibration zwischen inspirations- und exspirations Konzentration auszugehen, so daß bei einer standardmäßigen Äquilibierungszeit von 20 min. davon ausgegangen darf, daß die inspirations- und exspirations Konzentration identisch ist. Bei jeder Messung wurden PaO₂ (Partialdruck von Sauerstoff; tension in arterial blood), PaCO₂ (Partialdruck von Kohlendioxid; carbondioxid tension in arterial blood), pH-Wert, Natrium und Kalium mittels eines Blutgasanalysators (ABL 500 + EML 100, Radiometer Copenhagen, Danmark) bestimmt.
Die Harnblase wurde katheterisiert. Mit Beginn der Katheterisierung wurde bis zum Ende des Versuchs eine kontinuierliche Infusion einer Elektrolytlösung von 0,1 ml/kg KG/min begonnen. Nach Beendigung der Präparation wurde die Narkose mit Halothan solange fortgeführt, bis die Propofolwirkung keinen Einfluß auf den MAC-Wert hatte. Zur Bestimmung des Zeitpunkts dienten die Berechnungen nach Cockshott et al. und Adams et al., die beide zeigen konnten, daß die Propofol-But-Konzentration bei Boli-Gaben von 2-5 mg/kg KG (KG: Körpergewicht) nach 45 min auf unter 10 % der Ausgangskonzentration abgesunken war.

### Experimentelles Protokoll

Zur Bestimmungder MAC-Werte von Halothan in 0,15,30,40 und 50 und vol.% Xenon wurde die Konzentration des Halothans in Schritten um 0,1 Vol. % erhöht bzw. erniedrigt. Die 10 Tiere wurden randomisiert den je zwei Versuchsgruppen zugeteilt. Gruppe 1 begann mit Xenon 0 Vol.-% und absteigenden Konzentrationen des Halothans. Generell wurde frühstens 3 Stunden nach Prämedikation und 1 Stunde nach der letzten Propofol-Gabe mit dem Versuchsprotokoll begonnen. Hierdurch ließen sich Einflüsse der Prämedikation und der Propofolgaben ausschließen. Bis zum nächsten Messzeitpunkt wurde eine Äquilibrierungszeit von mindestens 15 min. eingehalten und abgewartet bis sich steadystate-Bedingungen eingestellt hatten. Die Messungen wurden solange durchgeführt, bis eine Änderung der Reaktion auf den Schmerzreiz zu verzeichnen war.

### Messung

Ein supramaximaler Schmerzstimulus wurde nach der dew-claw-clamp Methode von Eger et al. (1988) durchgeführt.

### Ergebnisse

Die Ergebnisse der einzelnen Xenon-Konzentrationen sind in Tab. 6 dargestellt.

**Tabelle 6: Dargestellt ist jeweils der Median mit dem 95 % Konfidenz-Intervall (mean: Mittelwert; SEM: Standard error of the mean).**

| | Probitanalyse | | Empirische Daten |
|---|---|---|---|
| Xenonconcentration (Vol. %) | MACₕₐₗₒ Median (Vol. %) | MACₕₐₗₒ bei 95 % Konfidenz (Vol. %) | MACₕₐₗₒ mean ± SEM |
| 0 | 1,02 | 0,95-1,09 | 1,08 ± 0,07 |
| 15 | 0,88 | 0,81-0,94 | 0,93 ± 0,07 |
| 30 | 0,81 | 0,75-0,87 | 0,87 ± 0,06 |
| 40 | 0,69 | 0,62 - 0,75 | 0,75 ± 0,06 |
| 50 | 0,65 | 0,58-0,72 | 0,71 ± 0,07 |

### Gasparameter, Hämodynamik und Elektrolyte

Während des gesamten Versuchsablaufs wurden die gemonitorten Parameter möglichst konstant gehalten. Es zeigen sich zwischen den unterschiedlichen Xenon-Konzentrationen keine signifikanten Unterschiede. Einzig der PaO₂ veränderte sich entsprechend des FiO₂ (fractional inspired oxygen tension). Durch Bestimmung des PaO₂/ FiO₂ Indexes lassen sich die Werte jedoch gut miteinander Vergleichen (s. Tab. 7 und 8).

**Tab. 7: Dargestellt sind die Meßergebnisse bei verschiedenen Xenon-Konzentrationen als mean ± SD (Mittelwert Standardabweichung).**

| Xenon (Vol.-%) | Temperatur (°C) mean ± SD | PaO₂ (mmHg) mean ± SD | PaO₂/FiO₂ Index | PaCO₂ (mmHg) mean ± SD | pH mean ± SD |
|---|---|---|---|---|---|
| 0 | 38,1 ± 0,6 | 459 ± 41 | 483 | 43 ± 2 | 7,48 ± 0,03 |
| 15 | 38,3 ± 0,5 | 383 ± 35 | 479 | 44 ± 2 | 7,48 ± 0,02 |
| 30 | 38,3 ± 0,4 | 331 ± 41 | 509 | 44 ± 2 | 7,49 ± 0,03 |
| 40 | 38,3 ± 0,4 | 277 ± 29 | 504 | 43 ± 2 | 7,48 ± 0,03 |
| 50 | 38,3 ± 0,5 | 226 ± 16 | 502 | 43 ± 2 | 7,48 ± 0,02 |

**Tab. 8: Dargestellt sind HR und MAP beivon Xenon 0 bis 50 Vol.-% als mean ± SD (Mittelwert ± Standardabweichung).**

| Xenon (Vol.%). | HR (min⁻¹) mean ± SD | MAP (mmHg) mean ± SD | Natrium mean ± SD | Kalium mean ± SD |
|---|---|---|---|---|
| 0 | 91 ± 14 | 62 ± 11 | 138 ± 1,5 | 4,5 ± 0,9 |
| 15 | 88 ± 10 | 65 ± 10 | 138 ± 2,0 | 4,6 ± 0,9 |
| 30 | 89 ± 9 | 70 ± 13 | 138 ± 1,8 | 4,5 ± 0,9 |
| 40 | 90 ± 10 | 72 ± 14 | 138 ± 1,6 | 4,4 ± 0,6 |
| 50 | 91 ± 9 | 74 ± 14 | 139 ± 1,5 | 4,0 ± 0,4 |

### 2. Durchführung der Narkose mit Isofluran-Xenon am Schwein

Die Versuche mit Isofluran-Xenon am Schwein wurden analog zu Beispiel 1 durchgeführt.

Die Ergebnisse sind in den folgenden Tabellen zusammengestellt.

**Tabelle 9: Meßergebnisse (mean SEM; SpO₂: Sauerstoftsättigung; BE: Basenüberschuß, base excess)**

| **Parameter** | **Xenon 50 Vol.-%** | **Xenon 40 Vol.-%** |
|---|---|---|
| **HR[min⁻¹]** | 100 ± 4,6 | 97,6 ± 3,66 |
| **MAP[mmHg]** | 75,4 ± 3,48 | 72 ± 3,15 |
| **Temp[°C]** | 38,9 ± 0,31 | 39,2 ± 0,25 |
| **Na⁺[mmol/l**] | 136 ± 0,54 | 135,7 ± 0,47 |
| **K⁺[mmoh/l]** | 4,26 ± 0,17 | 4,63 ± 0,14 |
| **CI⁻[mmol/l]** | 97,5 ± 0,83 | 98 ± 0,67 |
| **Glucose[mmol/l]** | 4,39 ± 0,43 | 4,8 ± 0,34 |
| **SpO₂[%]** | 99,4 ± 0,22 | 99,4 ± 0,22 |
| **PaO₂[mmHg]** | 228,9 ± 3,74 | 289,39 ± 17,4 |
| **PaCO₂[mmHg]** | 38,49 ± 0,56 | 38,55 ± 1,15 |
| **BE[mmol/l]** | 5,98 ± 0,62 | 6,4 ± 0,59 |
| **pH** | 7,48 ± 0,01 | 7,49 ± 0,01 |
| **HCO₃[mmol/l]** | 29,46 ± 0,6 | 29,83 ± 0,58 |
| **Lactat[mmol/l]** | 0,96 ± 0,1 | 0,91 ± 0,1 |

**Tabelle 10: Meßergebnisse (mean ± SEM)**

| **Parameter** | **Xenon 30 Vol.-%** | **Xenon 15 Vol.-%** | **Xenon 0 Vol.-%** |
|---|---|---|---|
| **HR[min⁻¹].** | 94,8 ± 2,66 | 93 ± 3,14 | 95,3 ± 4,73 |
| **MAP[mmHg]** | 70,6 ± 3,5 | 73,8 ± 5,35 | 73,6 ± 4,66 |
| **Temp[°C]** | 39,28 ± 0,21 | 38,9 ± 0,16 | 38,6 ± 0,29 |
| **Na⁺[mmol/]** | 136 ± 0,44 | 136 ± 0,33 | 136 ± 0,42 |
| **K⁺[mmol/l]** | 4,65 ± 0,14 | 4,53 ± 0,1 | 4,26 ± 0,22 |
| **Cl[mmol/l]** | 97±0,87 | 97,3 ± 0,73 | 97 ± 0,95 |
| **Glucose[mmol/l]** | 5,02 ± 0,17 | 4,86 ± 0,18 | 4,81 ± 0,2 |
| **SpO₂[%]** | 99 ± 0,21 | 99,4 ± 0,16 | 99 ± 0,15 |
| **PaO₂[mmHg]** | 347 ± 8,09 | 441,85 ± 11,6 | 498,42 ± 13,3 |
| **PaCO₂[mmHg]** | 37,88 ± 0,83 | 38,01 ± 1,13 | 37,92 ± 1,17 |
| **BE[mmol/l]** | 7,3 ± 0,67 | 6,82 ± 0,68 | 6,68 ± 0,49 |
| **pH** | 7,49 ± 0,01 | 7,49 ± 0,02 | 7,5 ± 0,02 |
| **HCO₃[mmol/l]** | 30,64 ± 0,62 | 30,19 ± 0,65 | 30,03 ± 0,42 |
| **Lactat[mmol/l]** | 0,92 ± 0,1 | 0,9 ± 0,1 | 0,83 ± 0,06 |

**Tabelle 11: Gemessene Parameter (Mittelwerte; mean ± SEM)**

| **Parameter** | **Xenon 50 Vol.-%** | **Xenon 40 Vol.-%** |
|---|---|---|
| PaO₂[mmHg] | 228,9±3,74 | 289,39 ±17,4 |
| PaCO₂[mmHg] | 38,49 ± 0,56 | 38,55 ± 1,15 |
| BE[mmol/l] | 5.98 ± 0,62 | 6,4 ± 0,59 |
| pH | 7,48 ± 0,01 | 7,49 ± 0,01 |
| HCO₃[mmol/l] | 29,46 ± 0,6 | 29,83 ± 0,58 |
| Lactat[mmol/l] 0,98 | 0,07 0,96 ± 0,1 | 0,91 ± 0,1 |

**Tabelle 12: Gemessene Parameter (Mittelwerte; mean SEM)**

| **Parameter** | **Xenon 30 Vol.-%** | **Xenon 15 Vol.-%** | **Xenon 0 Vol.-%** |
|---|---|---|---|
| PaO₂[mmHg] | 347 ± 8,09 | 441,85 ± 11,6 | 498,42 ± 13,3 |
| PaCO₂[mmHg] | 37,88 ± 0,83 | 38,01 ± 1,13 | 37,92 ± 1,17 |
| BE[mmol/l] | 7,3 ± 0,67 | 6,82 ± 0,68 | 6,68 ± 0,49 |
| pH | 7.49 ± 0,01 | 7,49 ± 0,02 | 7,5 ± 0,02 |
| HCO₃[mmol/l] | 30,64 ± 0,62 | 30,19 ± 0,65 | 30,03 ± 0,42 |
| Lactat[mmol/l] | 0.92 ± 0,1 | 0,9 ± 0,1 | 0,83 ± 0,06 |

**Tabelle 13: Isofluran-MAC-Wert in Abhängigkeit der inhalierten Xenon-Konzentration, berechnet nach Probitanalyse (median with 95% confidence limits)**

| Xenon [Vol.-%] | Isofluran MAC [Vol.-%] | untere Grenze [Vol.-%] | obere Grenze [Vol.-%] | Abnahme des MAC_{Iso} im Vergleich zu Vol.-% O₂ | Abnahme des MACᵢₛₒ per 10% 100 Xenon |
|---|---|---|---|---|---|
| Xenon 50% | 1,29 | 1,18 | 1,40 | 28,0% | 0,1 |
| Xenon 40 % | 1,55 | 1,44 | 1,68 | 13,8 % | 0,07 |
| Xenon 30 % | 1,68 | 1,58 | 1,79 | 8,5% | 0,05 |
| Xenon 15 % | 1,78 | 1,69 | 1,88 | 3,2 % | 0,04 |
| Xenon 0 % | 1,82 | 1,72 | 1,91 | | |

## Patentansprüche

1. Anästhesiemittel, enthaltend Sauerstoff, Xenon oder ein xenonhaltiges Gas und ein volatiles Anästhesiemittel als Kombinationspräparat zur gleichzeitigen, getrennten oder zeitlich abgestuften Anwendung bei der Anästhesie, **dadurch gekennzeichnet, dass** das Anästhesiemittel Xenon in einer Konzentration, in einem Bereich von 10 bis 50 Vol.-% enthält.

2. Anästhesiemittel nach Anspruch 1, **dadurch gekennzeichnet, dass** das volatile Anästhesiemittel Halothan, Enfluran, Isofluran, Sevofluran oder Desfluran ist.

3. Anästhesiemittel nach Anspruch 1 oder 2, **dadurch gekennzeichnet, dass** das volatile Anästhesiemittel Halothan, Enfluran, Isofluran, Sevofluran oder Desfluran ist und in dem Konzentrationsbereich von 0,2 bis 0,75 Vol.-% bei Halothan, von 0,6 bis 1,6 Vol.-% bei Enfluran, von 0,5 bis 1,15 Vol.-% bei Isofluran, von 0,9 bis 2,0 Vol.-% bei Sevofluran und von 3,6 bis 6,0 Vol.-% bei Desfluran eingesetzt wird.

4. Anästhesiemittel nach einem der Ansprüche 1 bis 3, **dadurch gekennzeichnet, dass** der Anteil von Xenon und volatilem Anästhesiemittel so bemessen ist, dass der Blutdruck des Patienten im Wesentlichen nicht verändert wird.

5. Anästhesiemittel nach Anspruch 1, **dadurch gekennzeichnet, dass** der Anteil von Sauerstoff mindestens 30 Vol.-% beträgt.

6. Anästhesiemittel nach Anspruch 1, **dadurch gekennzeichnet, dass** der Anteil von Sauerstoff mindestens 50 Vol.-% beträgt.

7. Verwendung von Sauerstoff, Xenon in einer Konzentration in einem Bereich von 10 bis 50 vol.-% und einem volatilen Anästhesiemittel zur Herstellung eines Kombinationsanästhesiemittels zur gleichzeitigen, getrennten oder zeitlich abgestuften Anwendung der Komponenten bei der Anästhesie.

8. Verwendung von Sauerstoff, Xenon in einer Konzentration in einem Bereich von 10 bis 50 vol.-% und einem volatilen Anästhesiemittel zur Herstellung eines Kombinationsanästhesiemittels zur gleichzeitigen, getrennten oder zeitlich abgestuften Anwendung der Komponenten bei der Anästhesie von Patienten mitcardiopulmonalen Erkrankungen oder bei Operationen mit hohem Blutverlust.

## Claims

1. Anaesthetic containing oxygen, xenon or a xenon-containing gas and a volatile anaesthetic as combination product for simultaneous, separate or sequential application in anaesthesia, **characterized in that** the anaesthetic comprises xenon in a concentration in a range of 10 to 50% by volume.

2. Anaesthetic according to Claim 1, **characterized in that** the volatile anaesthetic is halothane, enflurane, isoflurane, sevoflurane or desflurane.

3. Anaesthetic according to Claim 1 or 2, **characterized in that** the volatile anaesthetic is halothane, enflurane, isoflurane, sevoflurane or desflurane and is used in the concentration range of 0.2 to 0.75% by volume of halothane, 0.6 to 1.6% by volume of enflurane, 0.5 to 1.15% by volume of isoflurane, 0.9 to 2.0% by volume of sevoflurane, and 3.6 to 6.0% by volume of desflurane.

4. Anaesthetic according to one of Claims 1 to 3, **characterized in that** the proportion of xenon and volatile anaesthetic is chosen such that the patient's blood pressure is not essentially changed.

5. Anaesthetic according to Claim 1, **characterized in that** the proportion of oxygen is at least 30% by volume.

6. Anaesthetic according to Claim 1, **characterized in that** the proportion of oxygen is at least 50% by volume.

7. Use of oxygen, xenon in a concentration in a range of 10 to 50% by volume and of a volatile anaesthetic for producing a combination anaesthetic for simultaneous, separate or sequential application of the components in anaesthesia.

8. Use of oxygen, xenon in a concentration in a range of 10 to 50% by volume and of a volatile anaesthetic for producing a combination anaesthetic for simultaneous, separate or sequential application of the components in anaesthesia of patients with cardiopulmonary diseases or in operations involving substantial loss of blood.

## Revendications

1. Anesthésique contenant de l'oxygène, du xénon ou un gaz contenant du xénon et un agent anesthésique volatil, sous forme d'une préparation consistant en une association de composants, pour l'utilisation simultanée, séparée ou échelonnée dans le temps lors de l'anesthésie, **caractérisé en ce que** l'anesthésique contient du xénon à une concentration dans une plage allant de 10 à 50% en volume.

2. Anesthésique selon la revendication 1, **caractérisé en ce que** l'agent anesthésique volatil est l'halothane, l'enflurane, l'isoflurane, le sévoflurane ou le desflurane.

3. Anesthésique selon la revendication 1 ou 2, **caractérisé en ce que** l'agent anesthésique volatil est l'halothane, l'enflurane, l'isoflurane, le sévoflurane ou le desflurane et est utilisé dans la plage de concentration allant de 0.2 à 0.75% en volume dans le cas de l'halothane, de 0.6 à 1.6% en volume dans le cas de l'enflurane, de 0,5 à 1,15 % en volume dans le cas de l'isoflurane, de 0,9 à 2,0 % en volume dans le cas du sévoflurane et de 3,6 à 6,0 % en volume dans le cas du desflurane.

4. Anesthésique selon l'une des revendications 1 à 3, **caractérisé en ce que** la proportion du xénon et de l'agent anesthésique volatil est choisie telle que la pression artérielle du patient n'est pratiquement pas changée.

5. Anesthésique selon la revendication 1, **caractérisé en ce que** la proportion d'oxygène est d'au moins 30 % en volume.

6. Anesthésique selon la revendication 1, **caractérisé en ce que** la proportion d'oxygène est d'au moins 50% en volume.

7. Utilisation d'oxygène, de xénon à une concentration dans une plage allant de 10 à 50% en volume et d'un agent anesthésique volatil pour la préparation d'un anesthésique consistant en une association de composants, pour l'utilisation simultanée, séparée ou échelonnée dans le temps des composants lors de l'anesthésie.

8. Utilisation, d'oxygène, de xénon à une concentration dans une plage allant de 10 à 50% en volume et d'un agent anesthésique volatil pour la préparation d'un anesthésique consistant en une association de composants, pour l'utilisation simultanée, séparée ou échelonnée dans le temps des composants lors de l'anesthésie de patients souffrant de maladies cardio-pulmonaires ou dans des opérations s'accompagnant d'une importante perte de sang.
